# EUROPEAN PATENT SPECIFICATION

(11) **EP 2 196 222 B1**
(45) Date of publication and mention of the grant of the patent: **23.04.2014**
(21) Application number: 10154938.4
(22) Date of filing: 20.10.2004
(51) Int. Cl.: A61K 51/04, A61K 51/08, A61P 35/00

(54) **Tricarbonyl complexes of rhenium comprising amino acids as bidentate ligands and their use as radiotherapeutic chemotoxic agents.**
Tricarbonylkomplexe des Rheniums beinhaltend einen zweizähnigen Aminosäure-Ligand und deren Anwendung als radiotherapeutisch chemotoxische Agentien.
Complexes tricarbonyle du Rhenium comprenant une acide aminé comme ligand bidentique et leurs utilisation en tant qu'agents radiothérapeutiques et chémotoxiques.

(30) Priority: 20.10.2003 EP 03078086
(43) Date of publication of application: 16.06.2010
(62) Divisional of application: 04790748.0
(73) Proprietor: Universität Zürich, 8057 Zürich (CH)
(72) Inventor: Alberto, Roger, CH-8057 Zurich (CH); Zobi, Fabio, CH-8052 Zurich (CH)
(74) Representative: Jones, Nicholas Andrew

(56) References cited:
- WO-A-02/087633
- WO-A-2004/022105
- WO-A-2004/097406
- SEVERIN K ET AL: "BIOORGANOMETALLIC CHEMISTRY-TRANSITION METAL COMPLEXES WITH ALPHA-AMINO ACIDS AND PEPTIDES" ANGEWANDTE CHEMIE. INTERNATIONAL EDITION, VCH VERLAG, WEINHEIM, DE, vol. 37, no. 12, 1 June 1998 (1998-06-01), pages 1635-1654, XP002925262 ISSN: 0570-0833
- EGLI A ET AL: "ORGANOMETALLIC 99M TC-AQUATION LABELS PEPTIDE TO AN UNPRECEDENTED HIGH SPECIFIC ACTIVITY" JOURNAL OF NUCLEAR MEDICINE, SOCIETY OF NUCLEAR MEDICINE, RESTON, VA, US, vol. 40, no. 11, 1 January 1999 (1999-01-01), pages 1913-1917, XP009075361 ISSN: 0161-5505
- DU J ET AL: "Technetium-99m labelling of glycosylated somatostatin-14" APPLIED RADIATION AND ISOTOPES, ELSEVIER, OXFORD, GB LNKD- DOI:10.1016/S0969-8043(01)00046-X, vol. 55, no. 2, 1 August 2001 (2001-08-01) , pages 181-187, XP004231602 ISSN: 0969-8043
- ZHANG J ET AL: "Tricarbonylrhenium(I) complexes of phosphine-derivatized amines, amino acids and a model peptide: structures, solution behavior and cytotoxicity" JOURNAL OF ORGANOMETALLIC CHEMISTRY, ELSEVIER-SEQUOIA S.A. LAUSANNE, CH, vol. 650, no. 1-2, 1 May 2002 (2002-05-01) , pages 123-132, XP004351213 ISSN: 0022-328X
- R. ALBERTO: "[Tc(CO)3] chemistry: a promising new concept for SPET" EUR J NUCL MED MOL IMAGING, vol. 30, September 2003 (2003-09), pages 1299-1302, XP008051330
- SCHIBLI R ET AL: "Influence of the denticity of ligand systems on the in vitro and in vivo behaviour of [99m]Tc(I)-tricarbonyl complexes: a hint for the future functionalisation of biomolecules" BIOCONJUGATE CHEMISTRY, AMERICAN CHEMICAL SOCIETY, WASHINGTON, US, vol. 11, no. 3, May 2000 (2000-05), pages 345-351, XP002218742 ISSN: 1043-1802
- MUNDWILER S. ET AL: "Preparation of no-carrier added technetium-99m complexes via metal assisted cleavage from a solid phase" BIOCONJUGATE CHEM, vol. 15, 16 December 2003 (2003-12-16), pages 195-202, XP008051331
- ALBERTO R ET AL: "BASIC AQUEOUS CHEMISTRY OF M(OH2)3(CO)3+(M=REM TC) DIRECTED TOWARDS RADIOPHARMACEUTICAL APPLICATION" COORDINATION CHEMISTRY REVIEWS, ELSEVIER SCIENCE, AMSTERDAM, NL, vol. 190-192, 1999, pages 901-919, XP001074720 ISSN: 0010-8545
- PIETZSCH H-J ET AL: "CHEMICAL AND BIOLOGICAL CHARACTERIZATION OF TECHNETIUM(I) AND RHENIUM(I) TRICARBONYL COMPLEXES WITH DITHIOETHER LIGANDS SERVING AS LINKERS FOR COUPLING THE TC(CO)3 AND RE(CO)3 MOIETIES TO BIOLOGICALLY ACTIVE MOLECULES" BIOCONJUGATE CHEMISTRY, AMERICAN CHEMICAL SOCIETY, WASHINGTON, US, vol. 11, 2000, pages 414-424, XP001119310 ISSN: 1043-1802
- ZOBI, F. ET AL: "Toward novel binding metal complexes: Structure and basic kinetic data of [M(9MeG)2(CH3OH)(CO)3]+ (M0 99TC, Re)" INORG. CHEM, vol. 42, 4 May 2003 (2003-05-04), pages 2818-2820, XP008051339
- BELLA LA R ET AL: "A [99m]Tc(I)-postlabelled high affinity bombesin analogue as a potential tumor imaging agent" BIOCONJUGATE CHEMISTRY, AMERICAN CHEMICAL SOCIETY, WASHINGTON, US, vol. 13, no. 3, 15 May 2002 (2002-05-15), pages 599-604, XP002218739 ISSN: 1043-1802
- MUNDWILER S. ET AL: "A new [2+1] mixed ligand concept based on [99mTc(OH2)3(CO)3]+: a basic study" DALTON TRANS, 2 April 2004 (2004-04-02), pages 1320-1328, XP008051349
- ZOBI F. ET AL: "Head-to-head (HH) and head-to-tail (HT) conformers of cis-bis guanine ligands bound to the [Re(CO)3]+ core" INORGANIC CHEMISTRY, vol. 43, 17 February 2004 (2004-02-17), pages 2087-2096, XP008051340
- IOGANSON A A: "Metal Carbonyl Complexes with Ligands of Biological Origin", RUSSIAN CHEMICAL REVIEWS (USPEKHI KHIMII), INSTITUTE OF PHYSICS PUBLISHING, BRISTOL, GB, vol. 54, no. 3, 1 January 1985 (1985-01-01), pages 277-292, XP001537343, ISSN: 0036-021X
- KUNZE S ET AL: "Vitamin B12 as a ligand for technetium and rhenium complexes", ANGEWANDTE CHEMIE. INTERNATIONAL EDITION, WILEY VCH VERLAG, WEINHEIM, vol. 43, no. 38, 27 September 2004 (2004-09-27), pages 5025-5029, XP002328039, ISSN: 1433-7851, DOI: 10.1002/ANIE.200460923

## Description

The present invention relates to metal tricarbonyl complexes and to their use in the preparation of a medicament for the treatment of cancer.

It is now generally accepted that the cytotoxicity of the leading anticancer drug cisplatin is due to the formation of 1, 2-intrastrand adducts between the N7 atoms of two adjacent guanine residues in DNA. The products of this interaction are d(GpG)cross-links and less frequently d(ApG).

Not only have these adducts been observed both *in vitro* and *in vivo*, but clinically inactive compounds fail to form such cross-links.

Early structure-activity relationship studies indicated that for any cis-PtA₂X₂ analogue of cisplatin (A₂ is two amines or a bidentate amine ligand and X is an anionic leaving group) the carrier amine ligand had to have at least: one proton for the drug to retain its anticancer activity. This observation, along with the realization that d(GpG) can assume different conformations around the metal core, led to the hypothesis that hydrogen bonding interactions between bound G ligands and the carrier amine of the drug were important for the stabilization of the DNA distortion induced by the intrastrand lesion. It was also demonstrated that the guanine 06 H-bonding to carrier amine ligand hydrogen is not important for the bases to assume a particular orientation around the metal center and it was hypothesized that the small size of the NH group rather than its hydrogen-bonding ability is important for the anticancer activity of the drug.

One of the major disadvantages of cisplatin are its severe toxic side effects due to nonspecificity of the drug and the relatively large amounts to be administered. The drug is unspecific in its interaction with DNA and virtually any base can be platinated. Furthermore, many malignant tumors develop resistance to the drug. Also the coordination sphere of the metal ion cannot be derivatized with targeting agents as the molecules thus obtained lose their activity. Therefore, much interest remains in synthesizing metal complexes that are capable of binding to DNA bases in a fashion similar to cisplatin but do not present the disadvantages listed above.

Future cancer therapy will inter *alia* consist of a combination of several drugs or several effects which complement each other. The inventors contemplated that such a combination can also consist of radiotherapy and chemotherapy and might bring along important therapeutic advantages to cure cancer. Such therapeutic strategies would be in particular versatile if radio- and chemotoxicity would be based on one single compound. It was, therefore, considered by the inventors that it is desirable to employ compounds that might function mechanistically as cisplatin derivatives, causing intrastrand linkages of DNA by coordination of the metal center to two purine bases, in combination with an inherent radioactivity of the metal center. Such a class of compounds would act to inhibit DNA transcription while delivering a highly localized radiation dose in the target tumor tissues. The molecules may also be precisely localized in the body by well-established imaging techniques, allowing an exact quantification of the amounts of agent in the target tissues.

Zhang et al discloses tricarbonylrhenium complexes of phosphine derivatized amines for use as cytotoxic agents (J. organometallic Chem., 2002 vol. 650, no. 1-2, p.123-132).

On the basis of these considerations it is the object of the present invention to provide novel transition-metal complexes which combine both properties.

It was found that the [M(CO)₃]⁺ core (M = Re, Tc) can bind oligonucleotides comprising a GG motif with good stability and can cause similar structural changes in DNA as cisplatin. This was unexpected because the skilled person would expect coordination of this core to DNA bases to result in sterically too crowded complexes to have good stability. It was furthermore found that the [M(CO)₃]⁺ core surrounded by a proper set of ligands is chemotoxic and when M is a radioactive isotope also radiotoxic.

The invention thus relates to the use of metal tricarbonyl compounds [M(CO)₃L₃]⁺ as defined in the claims, wherein M is rhenium and L is a ligand as defined, for the preparation of a chemotoxic and optionally radiotherapeutic medicament for the treatment of cancer. In case cold rhenium is used the medicament is chemotoxic. In the case of a radioactive metal the compound is also radiotherapeutic.

The invention relates in particular to the use of tricarbonyl compounds of the general formula [M(CO)₃L₃]⁺, wherein M is an isotope of rhenium (in particular Re(I)) and L is a ligand for the preparation of a medicament for the treatment of cancer that is both chemotoxic by causing intrastrand linkages in DNA and radiotoxic.

Disclosed is the use of the tricarbonyl compounds of the general formula: 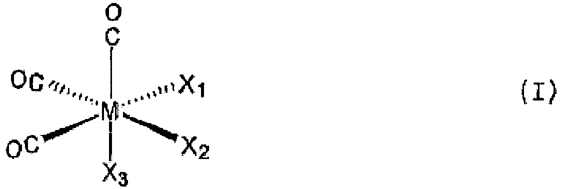 wherein
M is rhenium (Re (I)) or an isotope thereof; and
Xₐ, X₂ and X₃ is a monodentate ligand; or
two of X-L, X₂ and X₃ are such as to form compounds 4, 6, or 8-13.

Also disclosed are compounds of formulae 4, 6 or 8-13 as such. The following specification about the compounds thus relates to the compounds *per se*, as well as to the compounds of which the use is claimed.

The ligands serve two characteristics. First, they improve the rate and stability of binding to DNA. This concerns in particular the monodentate ligands. The compounds described may thus have one monodentate (for example complex 16), two monodentate (e.g. complex 18) or three monodentate ligands (e.g. complex 2). The presence of at least two monodentate ligands serves also to protect the [M(CO)₃]⁺ core from interacting with serum proteins. Such compounds are thus pro-drugs. In the intracellular space, these ligands are released and the drug is formed. A bidentate ligand serves exclusively protection. Complexes like 6, 10-13 are pro-drugs. The bidentate ligands are released and the compound becomes- active in cross-linking DNA. Compounds comprising exclusively mono- or bidentate ligands are unspecific (as is cisplatin) but linking a targeting biomolecule to either of them makes them target specific.

The monodentate ligands can be the same or different and can be selected from the group consisting of halogens, CO, aromatic heterocycles, thioethers, isocyanides. Aromatic heterocycles are five- or six-membered aromatic rings in which one or more of the members of the ring is an element other than C, e.g. N, S, O, P and mutual combinations thereof.

Within this group the halogens are selected from the group consisting of bromo, iodo, fluoro, chloro. Examples of suitable aromatic heterocycles are selected from the group consisting of pyridine, pyrimidine, pyrazine, imidazole, pyrazole, triazole, tetrazole, thiazole, oxazole and organic molecules having one of this group as an integral part. Suitable examples of thioethers are selected from the group consisting of linear substituted dialkyl-thioethers or cyclic thioethers such as tetrahydrothiophen and other organic molecules containing a thioether functionality as an integral part of it, and examples of suitable isocyanides are selected from organic molecules comprising a terminal -NC group coupled to an alkyl chain optionally comprising a terminal functionality such as a -COOH, -NH₂, -X, -SH, -OH group. Each one of the halogens can be combined with the same one or two-halogens or with each one of the aromatic heterocycles and/or with each one of the thioethers and/or each one of the isocyanides.

Each one of the monodentate ligands can be part of a larger molecule. For example, imidazole can be the side chain of a histidine in a peptide. The peptide in turn can be a targeting peptide.

The compounds disclosed comprise a bidentate ligand which can be selected from amino acids and dicarboxylates.

In a particular embodiment the bidentate ligand is an anionic amino acid. The advantage thereof is that amino acids are cleaved from the Re(I)- center at lower pH as encountered e.g. in cancer cells and lysosomes, thus, releasing the active part of the complex as a drug. Suitably, the amino acid is a non-natural α- or β-amino acid. In a particularly useful embodiment the non-natural amino acid is N,N-dimethyl glycine. While not wishing to be bound by theory, it is believed that since the two methyl groups are sterically demanding and the ligand is weaker bound to Re(I) than unmethylated glycine, this entails easier release at lower pH.

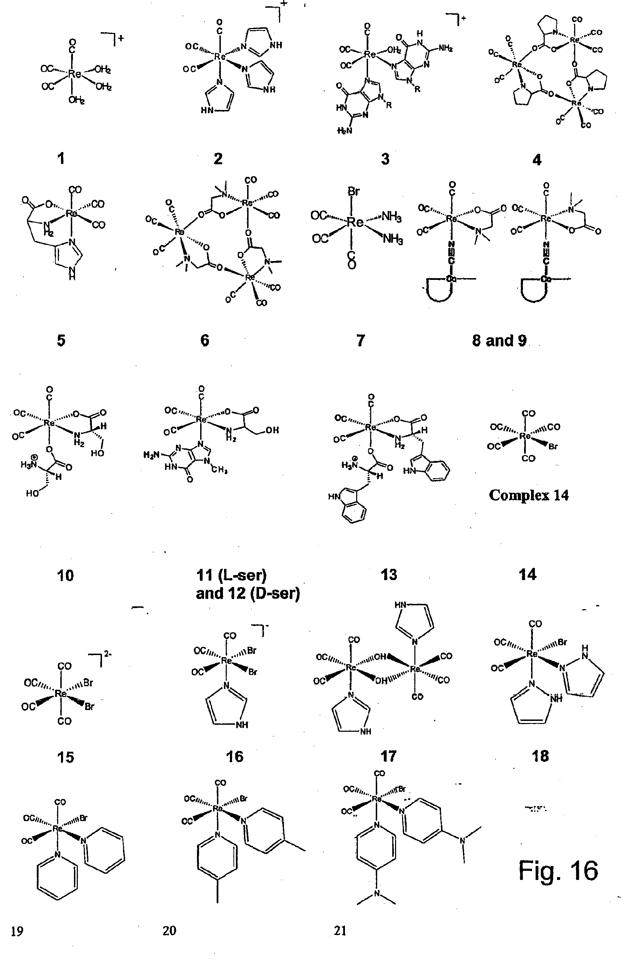

In a specific embodiment a disclosed compound is a complex selected from complexes 6, 10, 11, 12 and 13 as depicted in **Figure 16**.

Compounds of general formula I above are considered to have the required chemotoxic activity if they meet the following criteria. If at least two of the ligands in a compound as shown in formula I have been exchanged by guanine or guanosine after 3 days at 3.7°C with guanine or guanosine being preseat in a slight excess over rhenium, the starting complex is considered to have the claimed utility for cancer treatment.

The compounds disclosed can be derivatized in the sense that X₁ and/or X₂ and/or X₃ are coupled to a targeting moiety. Targeting moieties are known in the art and the skilled person is very well capable of selecting a targeting moiety that meets his needs. Suitable examples of targeting moieties are bombesin, neurotensin, somatostatin, glucosamine, nucleosides, nuclear localizing sequence peptides (NLS-peptides) oligonucleotides, nucleus targeting molecules such as anthracyclines, acridines and other intercalators.

The compounds herein described and used in accordance with the invention are based on mono-nuclear octahedral complexes of metal ions which combine the inherent radioactivity of the metal center with the mechanistic properties of cisplatin. This is unexpected since octahedral complexes are in general believed to be sterically too crowded to interact with DNA in a comparable way. Despite that, the present inventors have demonstrated that two nucleo-purines bind the Re(I) center in cis arrangement and do so at a rate comparable to that of platinum compounds leading to a chemotoxic activity comparable to cisplatin.

X-ray structures (see **Example 4**) of technetium and rhenium complexes bound specifically to two guanines via the N7 atoms together with kinetic and thermodynamic data of the interaction of [M(CO)₃(H₂O)₃]⁺ (wherein M = Re, Tc and isotopes thereof) with G and 2dG experimentally prove the intended structural properties. Correspondingly, comparison of these data with those of [Pt(NH₃)₂(H₂O)₂]²⁺, shows that 1 and 2 are potential chemotoxic agents affecting DNA like cisplatin. The radiotoxic mode of action of Re-186/188 is well established. As in the Pt case, two guanine ligands can adopt several conformations in an octahedral [(CO)₃Re(I)(purine)₂X] complex (X = H₂O, Br).

It is also shown by the present inventors that rhenium complexes with at least two available coordination sites influence the tertiary structure of ΦX174 DNA by altering the electrophoretic mobility of the open circular and the supercoiled form of plasmid DNA. The [Re(I)(CO)₃]⁺ moiety displays a principally similar reactivity pattern with plasmid DNA as e.g. cisplatin. It binds selectively to two free guanines, implying a possible interaction with adjacent guanines in DNA as well. The induced changes involve covalent binding to two bases rather than simple electrostatic interaction.

Furthermore, it was shown now that at a 200 µM concentration rhenium complexes are capable of inhibiting proliferation of certain types of human cancer cell lines. The improvements of the above mentioned compounds over the current state of the art are the following. Mono-nuclear octahedral ¹⁸⁶Re(I) or ¹⁸⁸Re(I) complexes can combine the radioactivity of the metal center with the ability off intra- or interstrand linking in DNA. Such a class of compounds can inhibit DNA transcription while delivering a highly localized radiation dose in the target tumor tissues. This type of complex can thus act as chemotoxic radiopharmaceuticals suitable for cancer therapy.

Compounds of the invention can easily be combined with vectors (i.e. polypeptides) that allow targeting, active uptake and degradation in the cytoplasm. A targeting biomolecule might be attached to X₁ or X₂ or X₃, or X₂/X₃ might be part of a larger structure, e.g. the imidazole side chain of histidine in a peptide or a GG motiv in an oligonucleotide. In the latter case, the GG motif protects the Re(I) active core but is released after oligonucleotide degradation in the cytoplasm.

Furthermore, non-radioactive substances with a structure identical to the radioactive ones can be added (tracer addition) to improve the therapeutic efficacy while the analogous radioactive compounds allow monitoring biodistribution. This is not possible with any current, metal based or organic chemotoxic agent in clinical use.

As it is well known that by combining radiotherapy and chemotherapy, important therapeutic advantages can be obtained to cure cancer, the above mentioned molecules are the first example of a molecular species comprising both properties (i.e. radioactivity and chemotoxicity) in one molecule.

Contrary to most other strategies which result in the design of Re (I) based compounds exclusively suited for radiotherapeutic purposes where the metal core is prevented from interacting further at the target site, the compounds of the invention can, upon delivery, actively participate in the biochemistry at the desired target tumor site.

The invention further allows for systematic drug discovery. By varying the substituents X₁, X₂ and X₃ at different positions various types of compounds can be obtained without undue burden. As a consequence, the molecules may be fine-tuned towards their interaction with DNA bases. Of course the decisive test is if such compounds meet the activity criteria.

Also described are pro-drugs. In such compounds the ligands are released from the pro-drug to generate the active form of the drug e.g. when the pH decreases as in cancer cells. Suitable examples of pro-drugs are compounds of formula I wherein at least two of X₁, X₂ and X₃ are a monodentate ligand or part of a bidentate ligand as defined above. Furthermore, the pro-drug can be coupled to targeting agents or metabolically active substances which might increase the therapeutic index. The ligands on the pro-drugs can protect the [M(CO)₃]⁺ core and can dissociate and release the "active form" of the drug in cancer cells.

In the case of pro-drugs, in the compounds of formula I X₁ represents, for example, a monodentate ligand whereas X₂ and X₃ are monodentate ligands or form together a bidentate chelator. The definitions of X₁, X₂ and X₃ are as described above. X₂X₃ represent the protecting group ligand(s) forming the pro-drug which are released in the cell to form the drug. X₁ influences the efficacy of DNA binding and the release of the pro-drug.

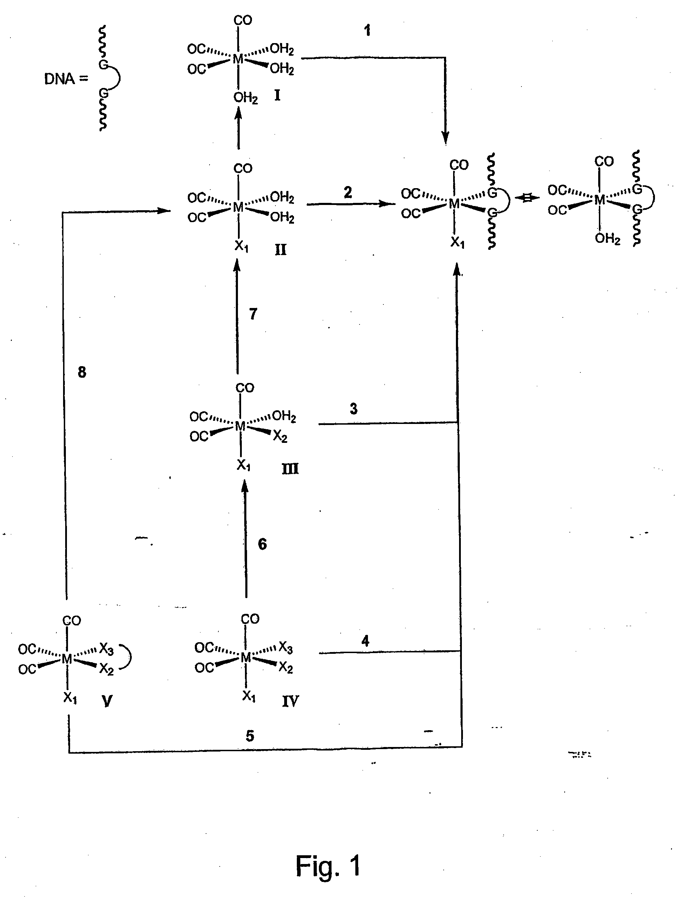

The different aspects of the disclosure are explained in **Figure 1**. In this figure compound I reacts with isolated G or DNA to form intra- or interstrand cross-links (**Examples 4**, **5** and **7**), compound II reacts with G or DNA to form intra- or interstrand cross-links (**Example 3** and **13**). Compounds I and II are considered as drugs since they also conjugate to serum proteins and can therefore not immediately been taken up by the cell, thus, they are inactive, compound III is an intermediate. It can directly react with DNA (drug) after being taken up in the cell. Since it does not strongly interact with serum proteins, it can be considered as a prodrug (**Example 13**, complex **8**) or it can lose one ligand, become compound II and act as a drug. The real pro-drugs are IV and V. Both do not react with serum proteins (hence they are pro-drugs) but could in principle directly react with DNA. More likely and shown in **Example 8** is the loss of ligands X₂ and X₃ to form compound II which is then the drug Pathways 1- 5 are drugs, pathways 6-8 are pro-drugs.

In this application the words "chemotoxic" and "cytotoxic" are used interchangeably. A compound is for example in itself chemotoxic but it has a cytotoxic effect on a cell. Cytotoxicity testing thus relates to the effect a compound will have on a cell, whereas chemotoxicity is an inherent feature of a compound. Furthermore, the words "compound" and "complex" are used interchangeably.

The present invention will be further elucidated in the Examples that follow. Reference is made to the following figures:
**Figure 1** shows a general scheme for the compounds and reaction pathways of the compounds claimed.
**Figure 2** shows the activity test for compounds 16, 16a and 3.
**Figure 3** shows the HPLC-MS chromatograph of **Example 3.**
**Figure 4** shows the X-ray crystal structures of [Re(9-MeG)₂(H₂O) (CO)₃] (ClO₄) and of [⁹⁹Tc (9-MeG) ₂ (CH₃OH) (CO)₃] (ClO₄).
**Figure 5A** shows the aromatic region (7.0-9.0 ppm) at the end of the reaction of 1 with d(CpGpG).
**Figure 5B** shows the pH dependence study confirming that the bis [Re (CO)₃d(CpGpG) (H₂O)] adduct binds to N7 atoms of guanine residues.
**Figure 6A** shows the action of cisplatin on ΦX 174 plasmid DNA. Lines 4-8 show the increased amount of scrambling as a consequence of cis-GG binding of cisplatin.
**Figure 6B** shows the interaction of complex 1 on ΦX174 plasmid DNA at conditions as described above. The result is similar to the one observed with cisplatin in **Figure 6A****.**
**Figure 6C** shows the interaction of the cationic Complex 2 with ΦX174 plasmid DNA. No scrambling of DNA is observed.
**Figure 7****,** lanes 2-7 show that the complexes with two labile cis ligands induce scrambling in ΦX174 plasmid DNA, whereas in lanes 8-14 no structural change is observed.
**Figure 8A** shows the interaction of ΦX174 plasmid DNA with complexes 1 and 6. Obviously, complex 6 causes DNA scrambling which is indicative for GG cross-links, comparable to cisplatin.
**Figure 8B** shows the structure of the pro-drug 6 and the resulting drug 1 and the X-ray structure of the pro-drug.
**Figure 8C** shows the schematized conversion of the pro-drug 6 to the active drug.
**Figure 9A** shows incubation of ΦX174 plasmid DNA with complexes **1** and **7** according to the procedure described in **Example 6.**
**Figure 9B** shows same samples after incubation with histidine to release the metal complexes and to reconstitute the original shape of ΦX174 plasmid DNA.
**Figure 10** shows a typical XTT cell proliferation assay for the determination of the cytotoxicity of the rhenium complexes as used in **Examples 10-13.**
**Figure 11** is a graphic representation of the cytotoxicity (% of cell survival) exhibited by complexes **1,** 3, **4** and **5** toward MDA-MB-4355 Breast Cancer Cells (ATCC #TB129).
**Figure 12** is a graphic representation of the cytotoxicity (% of cell survival) exhibited by complexes **1,** 3, **4** and **5** toward OVMZ-6-WT Ovarian Cancer Cells (obtainable from Deutsche Sammlung von Mikroorganismen und Zellinien DSMZ GmbH).
**Figure 13** is a graphic representation of the cytotoxicity (% of cell survival) exhibited by complexes **1,** 3, **4** and **5** toward HSC45-M2 Gastric Cancer Cells (Deutsche Sammlung von Mikroorganismen und Zellinien DSMZ GmbH)
**Figure 14A** is a graphic representation of the cytotoxicity (% of cell survival) exhibited by complexes **1** to **13** toward B16F1 mouse melanoma cells (Deutsche Sammlung von Mikroorganismen und Zellinien DSMZ GmbH)
**Figure 14B** is a graphic representation of the cytotoxicity (% of cell survival) exhibited by complexes **1, 2** and **14** to **21** toward B16F1 mouse melanoma cells (ATCC) compared to cisplatin.
**Figure 15** shows the coupling of the targeting moiety acridine to N-ethylamino-imidazole.
**Figure 16** is an overview of compounds 1 to 21.

### EXAMPLES

### EXAMPLE 1 (Comparative)

### Synthesis of compounds with a monodentate ligand

### 1. General method

The compounds of the invention with monodentate ligands generally can be synthesized by adding one equivalent of the ligand to a solution of 1.

### 2. Specific example ([Et₄N] [ReBr₂(Im) (CO)₃] (16))

As an example the synthetic procedure for 16 is given below: (Et₄N)₂[ReBr₃(CO)₃] (1, 96 mg, 0.12 mmol) was dissolved in CH₂Cl₂ (5 mL). Imidazole (Im, 8 mg, 0.12 mmol) was added. and the mixture was stirred at room temperature. After 30 min a white solid appeared. This was filtered and dried under vacuum. Yield,: 45 mg, 60%.

Elemental analysis calculated for 16, C₁₄H₂₄N₃0₃Br₂Re (628,38): C, 26.75; H, 3.82; N, 6.68, found: C, 26.83; H, 3.71; N, 6.62.

### EXAMPLE 2

### Synthesis of compounds of the invention with a bidentate ligand

### 1. General method

The compounds of the invention with bidentate ligands generally can be synthesized by adding one or more equivalents of the ligand to a solution of 1.

### 2. Specific example ([Re(L-Ser)₂(CO)₃] (10))

As an example the synthetic procedure for 10 is given below: (Et₄N)₂[ReBr₃(CO)₃] (100 mg, 0.13 mmol) was dissolved in a methanol/water mixture (9:1, 5 mL) . L-serine (48 mg, 0.46 mmol) was added and the mixture was stirred for 3 h at 50°C under a slight N₂ pressure. The reaction was monitored by HPLC and it was stopped when no further change could be observed (3h). The solution was allowed to equilibrate to room temperature and purified by HPLC. A white solid was obtained. Yield : 23 mg, 37%. Crystals suitable for x-ray analysis were 10 obtained by slow evaporation of H₂0.

Elemental analysis calculated for 10, C₉H₁₃N₂0₉Re (479,41): C, 22.55; H, 2.73; N, 5.84, found: C, 23.17; H, 3.20; N, 5.47.

### EXAMPLE 3 (Comparative)

### [M(CO)₃1⁺ binding to guanine

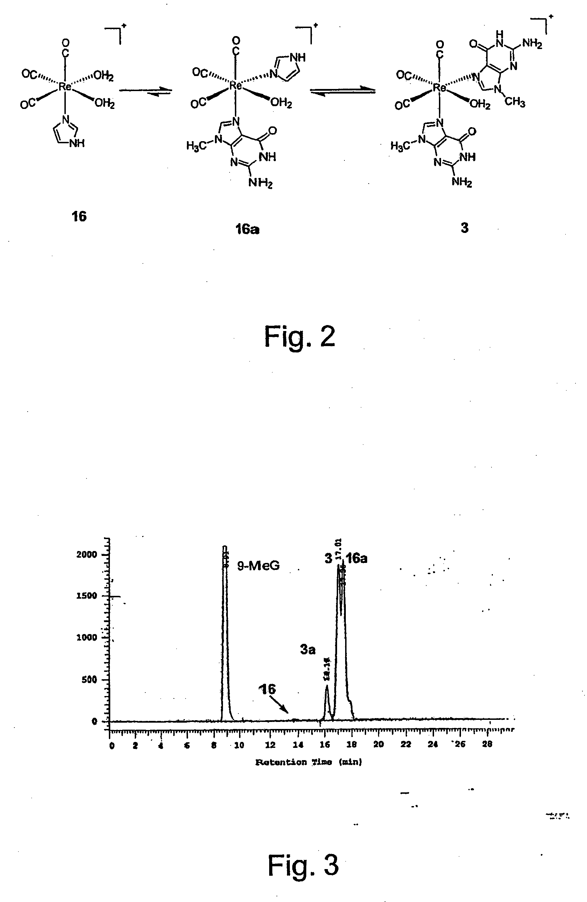

In order to test whether a metal tricarbonyl can bind to purine bases the following test was performed. A 1mM aqueous (or H₂O/CH₃OH mixture) solution of compounds of general formula I incubated at 37°C for 3 days with a 6-fold excess of guanine shows more than 50% binding of one or two guanines to the metal center **(****Figure 2****).**

In water, (37°C) **16** reacts with 9-MeG stepwise. In our HPLC gradient complex **16** has a retention time (rt) of 13.9 man. After 1h a second peak is observed with rt of 17.4 min. HPLC-MS chromatography indicates that this species is [Re(Im) (9-MeG) (H₂O) (CO)₃] **(16a)**. After a further 12h a third and a fourth peak appear at 17.0 and 16.2 min which were identified by HPLC-MS chromatography as [Re (9-MeG)₂ (H₂O) (CO₃)⁺ (3) and [Re(9-MeG) (H₂0)₂(CO)₃]⁺ (3a) respectively. The relative height of the peaks, with species 3 and 3a increasing in concentration, gave the only other change observed after a further 12h period of incubation (Figure 3).

This example shows, that guanines can substitute imidazole as a protecting ligand in compounds of general formula I. Compound 3 is a model for the structural feature of the [M(CO)₃]⁺ moiety after cross-linking guanines in DNA.

### EXAMPLE 4 (Comparative)

### Formation of a [M(CO)₃l⁺ (M = ⁹⁹Tc(I), Re (I)) bis guanine adduct [Re(9-MeG)₂(H₂O) (CO)₃] (ClO₄) (3)

(Et₄N)₂ [ReBr₃ (CO)₃] (30 mg, 0.04 mmol) was dissolved in hot (∼40°C) water (3 mL). AgClO₄ (28 mg, 0.14 mmol) was added and the mixture was stirred for 3 h after which time AgBr was filtered off. 9-methylguanine (16.5 mg, 0.1 mmol) was added and the mixture was heated to 50°C under a slight N₂ pressure. The colorless solution turned light yellow within minutes. The reaction was monitored by HPLC and it was stopped after 3.5 hr when no further change could be observed. The solution mixture was allowed to equilibrate to room temperature, concentrated and then purified on a short C18 column.

To the methanol fraction containing the purified complex 3% H₂0 (v/v) was added. Pentane was allowed to diffuse into the solution depositing x-ray quality crystals. Yield : quantitative.

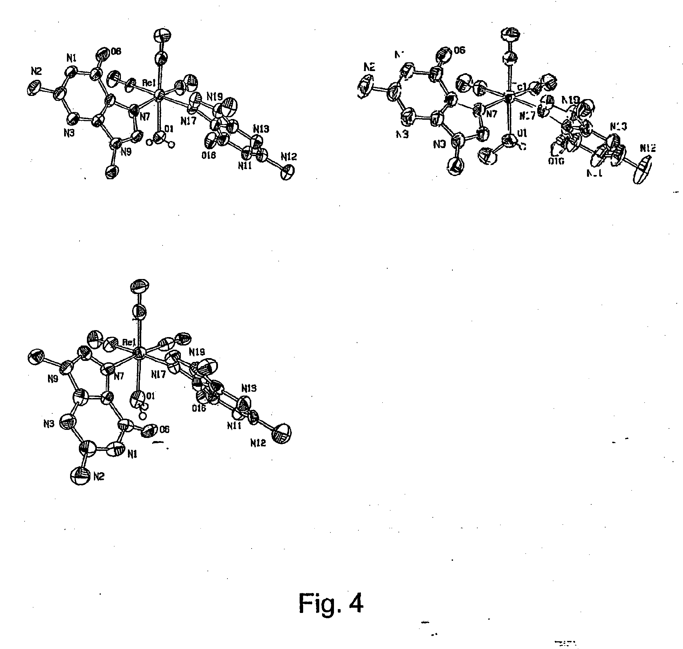

Elemental analysis calculated for 3, C₁₅H₁₆ClN₁₀O₁₀Re (718.01): C, 25.09; H, 2.25; N, 19.51, found: C, 25.34; H, 25 2.70; N, 19.45.
The X-ray crystal structure is shown in **Figure 4****.**

### EXAMPLE 5 (Comparative)

### Interaction of [M(CO)₃]⁺ with oligonucleotides

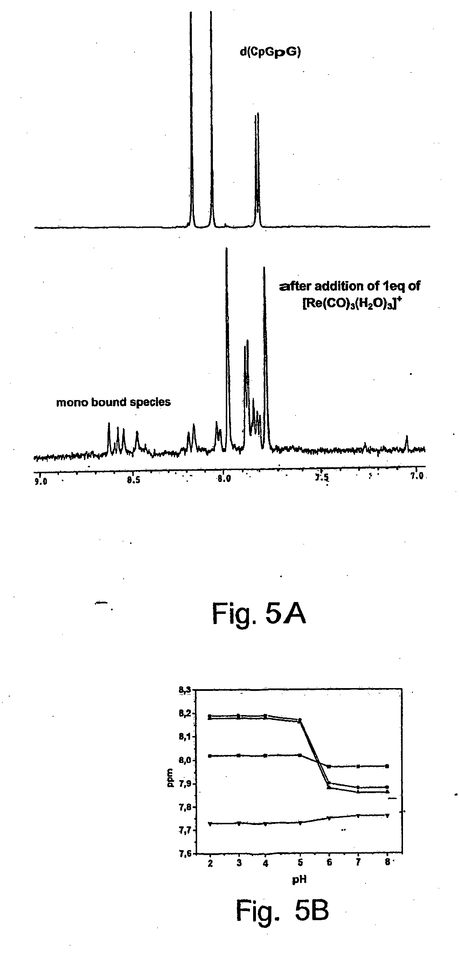

Figure 5A shows the ¹H NMR spectrum of the reaction of [Re(H₂0)₃(CO)₃]⁺(1) with 1 eq. of d(CpGpG) in D₂O. At 37°C the addition of 1 to a solution of d(CpGpG) causes the disappearance in the spectrum of the resonances due to the H8 signals of free d(CpGpG) and the appearance of a new set of sharp well separated peaks of the non equivalent H8 protons.

**Figure 5A** shows the aromatic region (7.0-9.0 ppm) at the end of the reaction of 1 with d(CpGpG) (Ih incubation).

The two guanine bases bind to Re(I) through N7, a fact corroborated by the pH independence of the H8 resonances at pH values near 2 **(****Figure 5B****).** In fact all chemical shifts of the H8 are unaffected by lowering the pH below 4, contrary to what is expected for a free guanine N7.

### EXAMPLE 6 (Comparative)

### Interaction of complexes 1 and 2 with ΦX 174 plasmid DNA

ΦX174 plasmids were purchased from Promega and used without further purification. ΦX174 RP plasmid DNA (0.1 mg) was mixed with the corresponding rhenium complexes in H₂O at [complex]/[bp] 0.018-1.8/1. The mixtures were incubated in water at 37°C for 22 h in the dark before analyzing by gel electrophoresis. The pH of the mixtures remained constant at ≈ 7 in all cases. Experiments performed in 1 mM or 10 mM NaClO₄ showed no significant difference in the binding of 1 to ΦK174 RF plasmid DNA.

DNA binding was examined by gel electrophoretic mobility shift assays through 9 cm 0.75% agarose slab gels with TAE running buffer. The gels were run at RT, with voltages varying between 50 and 75 V. Running time depended upon the voltage and were usually between 1.5-2 h. The resultant gels were stained with ethidium bromide in the buffer at a concentration of ≈0.3 ug/mL. Bands were visualized by software UV transillumination equipped with a digital camera.

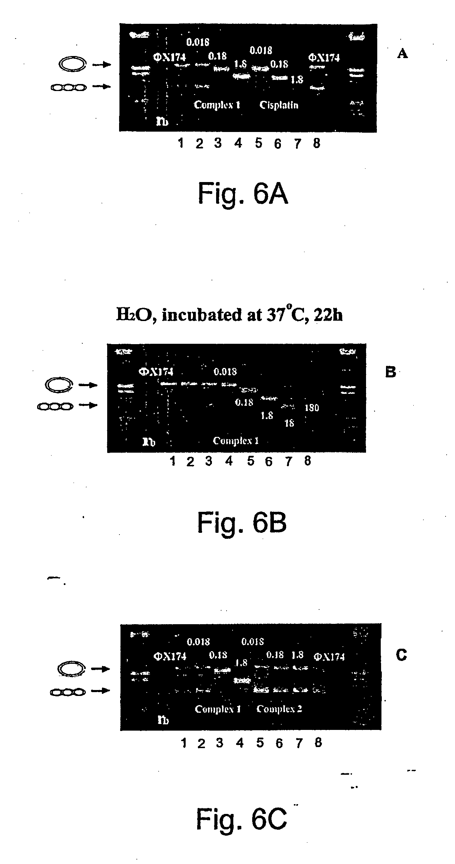

Figure 6A shows the action of cisplatin on ΦX174 plasmid DNA. Lines 4-8 show the increased amount of scrambling as a consequence of cis-GG binding of cisplatin.

**Figure 6B** shows the interaction of complex **1** on ΦX174 plasmid DNA at conditions as described above. The result is similar to the one observed with cisplatin in **Figure 6A****.**

**Figure 6C** shows the interaction of the cationic Complex **2** with ΦX174 plasmid DNA. No scrambling of DNA is observed.

It follows that [M(CO)₃]⁺ interacts with ΦX174 plasmid DNA in a fashion similar to cisplatin. It is also shown that the interaction is not due to electrostatic effects.

### EXAMPLE 7

### Two cis labile ligands are required to induce structural changes of ΦX174 plasmid DNA

In this example, ΦX174 plasmid DNA has been incubated with different complexes containing mono- or bidentate ligands. The complexes **1** and **3** comprise cis-labile ligands whereas complexes **4** and **5** are stable towards substitution with two G's.

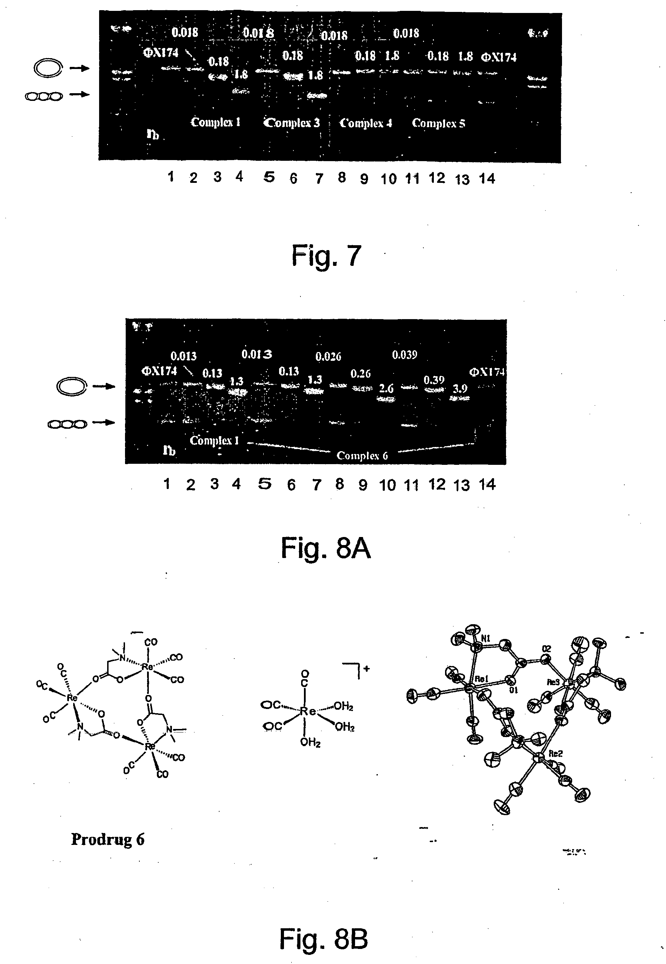

**Figure 7**, lanes 2-7 show that the complexes with two labile cis ligands induce scrambling in ΦX174 plasmid DNA, whereas in lanes 8-14 no structural change is observed. If the two cis labile ligands are only slowly released, then the precursor complex can be considered as a prodrug. This behavior is described in **Example 8.**

### EXAMPLE 8

### Preparation of a pro-drug

A pro-drug containing complex 1 as the effective drug contains two labile ligand in cis arrangement which are slowly released from the Re(I) center. After cleavage of the labile ligands a complex of the composition [M(X₁)(OH₂)₂(CO)₃] is formed which represents the active drug. We describe here the synthesis of such a pro-drug containing N,N-dimethyl-glycine as the cis-labile ligand.

Complex **1** (Et₄N)₂[ReBr₃(CO)₃] (100 mg, 0.13 mmol) was dissolved in a methanol/water mixture (4:1, 10 mL). N,N-dimethylglycine (70 mg, 0.7 mmol) was added and the mixture was stirred for 12 h at 50°C under a slight N₂ pressure. The solution was allowed to equilibrate to room temperature concentrated and purified on a short C18 filter. A white crystalline solid was obtained. Yield : 20 mg, 40%.

Crystals suitable for x-ray diffraction were obtained by slow diffusion of ether in a CH₃NC solution of the complex. Elemental analysis calculated for **6**, C₂₁H₂₄N₃O₁₅Re₃ (1117.05) : C, 22.58; H, 2.17; N, 3.76, found: C, 23.19; H, 2.78; N, 3.84. 1H NMR (500 MHz, DMSO-d6, d/ppm): 4.18 (s, 2H), 3.46 (s, 3H), 3.15 (s, 3H).

FT-IR for **6** (KBr, -ν/cm⁻¹): (C=O) 2022 (s), (C=O) 1911 (b), (C=O) 1890 (s), (C=O) 1866 (s). ESI-MS for 6 (ESI+, 40V, m/z) : 1117.0 ([M]⁺). HPLC Rₜ for **6** (HPLC, Gradient 1, min) : 15.7.

**Figure 8A** shows the interaction of ΦX174 plasmid DNA with complexes **1** and **6**. Obviously, complex **6** causes DNA scrambling which is indicative for GG cross-links, comparable to cisplatin.

**Figure 8B** shows the structure of the pro-drug **6** and the resulting drug **1** and the X-ray structure of the pro-drug.

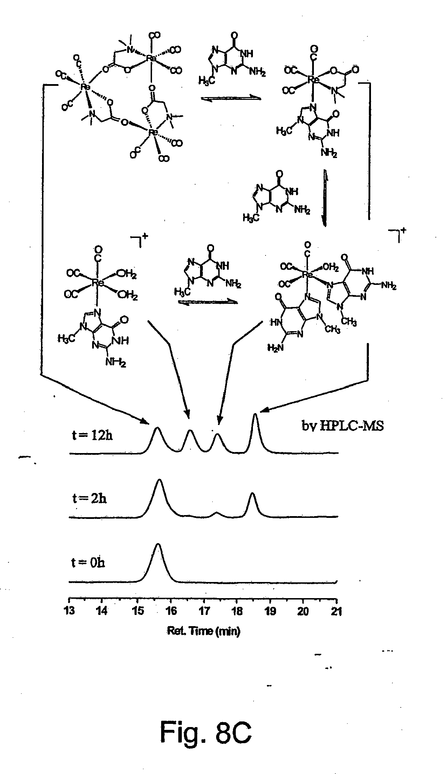

The interaction of complex **6** with guanine has been studied according to the test outlined in **Example 4**. NMR and HPLC experiments clearly show, that the bidentate ligand N,N-dimethyl-glycine is cleaved and replaced with two guanines, the same that happens in ΦX174 plasmid DNA. **Figure 8C** shows the schematized conversion of the pro-drug 6 to the active drug.

### EXAMPLE 9(Comparative)

### Stability of the [M(CO)₃]⁺ ΦX174 plasmid DNA adduct

As outlined in **Example 6,** complexes with cis-labile ligands can bind to ΦX174 plasmid DNA, presumably through GG inter- or intrastrand cross-links. In case of cisplatin binding to DNA, this interaction is irreversible. *In vitro* studies with the complexes **1** and **7** have been performed to assess this stability for two examples of compounds claimed inhere.

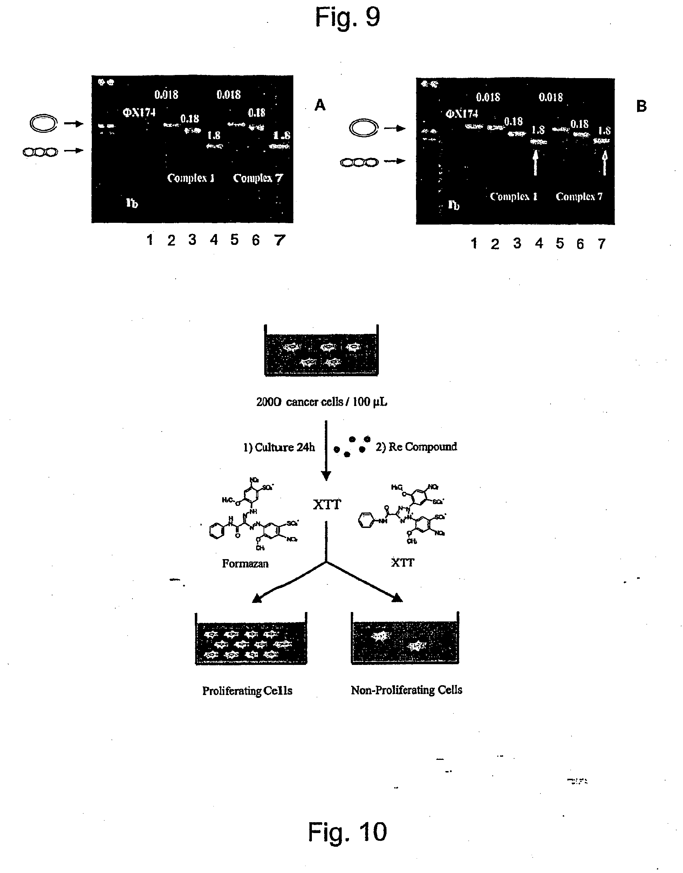

Complexes 1 and 7 were incubated with ΦX174 plasmid DNA as described in **Example 6.** Subsequently, the plasmid was challenged with histidine to cleave the complexes from ΦX174 plasmid DNA. Although a 100-fold excess of histidine was employed, no release could be observed, i.e. the structural changes in ΦX174 plasmid DNA could not be reversed. The gel electrophoresis traces after 22h are shown in **Figure 9A****.**

**Figure 9A** shows incubation of ΦX174 plasmid DNA with complexes **1** and **7** according to the procedure described in **Example 6.** **Figure 9B** shows same samples after incubation with histidine to release the metal complexes and to reconstitute the original, shape of ΦX174 plasmid DNA.

### EXAMPLE 10

### Cytotoxicity Procedure

In a typical experiment (see **Figure 10**) an average of 2000 cells were grown in microtiter plates (tissue culture grade, 96 wells, flat bottom) in a final volume of 100 µL culture medium per well in a humidified-atmosphere (37°C, >6.5% CO2). After 24 h the rhenium complex was added to the wells (final concentrations 200 µM based on Re) and the cells were grown for further 24 h under a humidified atmosphere. After the incubation period 50 µL of the XTT labeling mixture were added to each well. The plates were incubated again for 4 h. After the this final incubation period the spectrophotometrical absorbance (optical density OD) of each well was measured at 450 nm.

Control experiments were performed as described above without the addition of the rhenium compounds. Blanks were obtained by adding 50 µL of H₂O instead of the XTT labeling mixture. Experiments were done in double and the results represent the average.

The % of cell survival was calculated base on the relative OD of the samples. Maximum control OD was set to 100% cell survival.

### EXAMPLE 11

### Cytotoxicity of [Re(I)(CO)₃]+ complexes (200 µM) towards MDA-MB-4355 Breast Cancer cell line

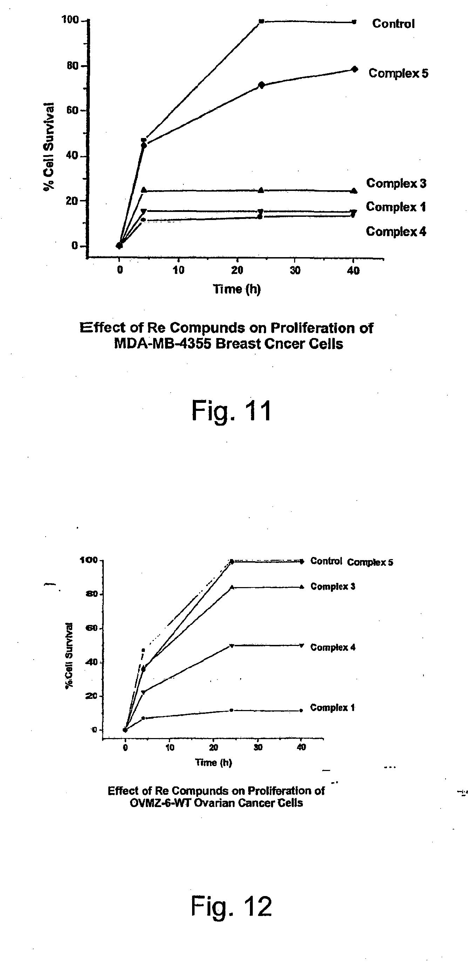

**Figure 11** shows a graphic representation of the cytotoxicity (% of cell survival) exhibited by complexes **1, 3, 4** and **5** toward MDA-MB-4355 Breast Cancer Cells. The results clearly indicate that compounds 1**, 3** and **4** inhibit cell proliferation. Complex **5** is stable under the conditions indicated in **Example 3** and does not show cytotoxicity towards this breast cancer cell line.

### EXAMPLE 12

### Cytotoxicity of [Re(I)(CO)₃]⁺ complexes (200 µM) towards OVMZ-6-WT Ovarian Cancer cell line

The procedure is as described in **Example 10** and **Figure 10****.** Only the cancer cell line varies.

**Figure 12** shows a graphic representation of the cytotoxicity (i.e. % of cell survival) exhibited by complexes **1, 3, 4** and **5** toward OVMZ-6-WT Ovarian Cancer Cells. The results clearly indicate that compound **1** inhibits cell proliferation in this cancer cell line.

### EXAMPLE 13

### Cytotoxicity of [Re(I)(CO)₃]⁺ complexes (200 µM) towards HSC45-M2 Gastric Cancer cell line

The procedure is as described in **Example 10** and **Figure 10**. Only the cancer cell line varies.

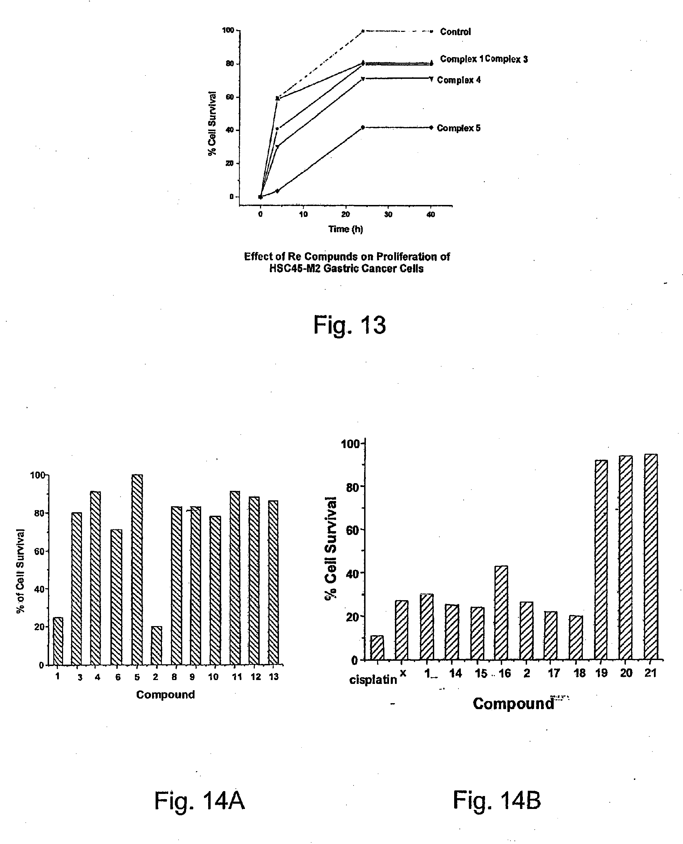

**Figure 13** shows a graphic representation of the cytotoxicity (i.e. % of cell survival) exhibited by complexes **1, 3, 4** and **5** toward HSC45-M2 Gastric Cancer Cells. The results clearly indicate that compound 5 inhibits cell proliferation in this cancer cell line.

### EXAMPLE 14

### Cytotoxicity of different rhenium complexes towards B16 F1 mouse melanoma cells

The procedure is as described in **Example 10** and **Figure 10**. Only the cancer cell line varies.

**Figure 14A** shows a graphic representation of the cytotoxicity (i.e. % of cell survival) exhibited by complexes **1** to **13** toward B16 F1 mouse melanoma cells. The results clearly indicate that compounds **1** and **2** strongly inhibit cell proliferation. Due to poor water solubility the concentration-of compounds **4, 5, 6** and **11** to **13** is lower than 200 mM. Consequently the above-mentioned compounds might show higher cytotoxicity at 200 mM.

**Figure 14B** shows a graphic representation of the cytotoxicity (i.e. % of cell survival) exhibited by complexes **1, 2** and **14** to **21** toward B16 F1 mouse melanoma cells. The results clearly indicate that compounds **1, 2** and **14** to **18** inhibit cell proliferation to an extent comparable to cisplatin.

### EXAMPLE 15 (Comparative)

### Coupling of the compounds of the invention to a targeting moiety

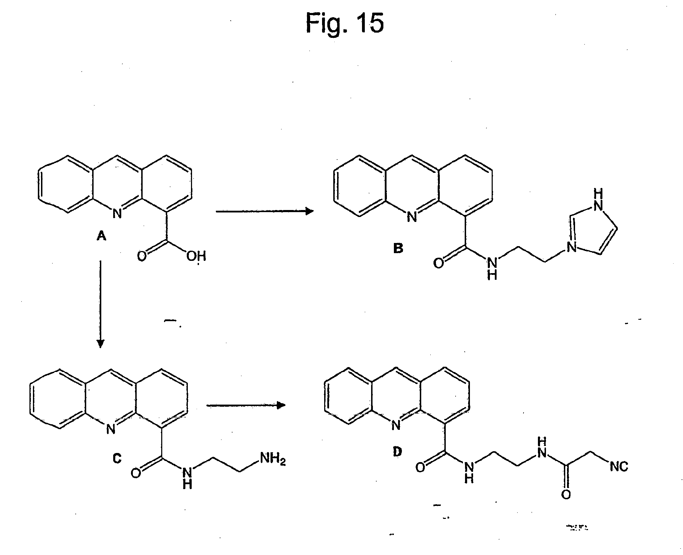

In this example, the targeting moiety represents acridine (A), a non-receptor binding but nucleus targeting organic molecule. Acridine has been derivatized with an isocyanide and an imidazole group for binding to the [M(co)₃]⁺ moiety. The basic and active structures are those of complexes **16** and **18,** both of which have been shown to crosslink ΦX174 plasmid DNA. The general reaction scheme for the preparation of the nucleus targeting agents is given in **Figure 15****.**

Compound A has been coupled to N-ethylamino-imidazole by standard coupling techniques. A was dissolved in THF and 15 activated with dicyclohexylcarbodiimide (DCC) and N-hydroxysuccinimide. After activation was complete, 0.9 eq. of the imidazole derivative was added and the mixture stirred for 12 h at RT. HPLC showed quantitative conversion of A → B. The compound was used without further purification.

Compound **A** was activated as described above and mixed with a 50-fold excess of 1,2-diamino-ethane in THF. The solution was allowed to stir overnight. The solvent was removed *in vacuo* and the residue washed several times with saturated NaHCO₃. The residue was dissolved in methanol and purified by column chromatography (silica gel, MeOH/CH₂Cl₂ 1/3 v/v). Compound C was then mixed with isocyano-acetic acid-ethylester and reacted for 48h. After this time C converted quantitatively to **D.** The reaction mixture was purified by column chromatography (silica gel, CH₂Cl₂/hexane 2/1).

One equivalent of compound **D** is now reacted with 1 to yield a compound [Re (D)Br₂ (CO)₃]⁻, a composition according to formula I.

### EXAMPLE 16 (Comparative)

### Monitoring the biodistribution of the compounds of the invention

The complexes of general formula I are synthesized with ^{99m}Tc(I) following the same procedures as outlined for rhenium. The starting complex **1** (^{99m}Tc(I)) is prepared from the Isolink Kit or according to a published procedure (Alberto et al. J. Am. Chem. Soc. 1999, 121(25), 6076-6077). The "cold" cytotoxic rhenium complexes are mixed with the "hot" radiotoxic ^{99m}Tc(I) or ^{188/186}Re(I) complexes. Since these complexes with either isotope of Tc(I) or Re(I) are analogues to each other, imaging with standard techniques SPECT cameras allows to follow where the compounds are accumulating.

Background to the invention may be found in following clauses:
1. Use of metal tricarbonyl compounds of the general formula [M(CO)₃L₃]⁺, wherein M is rhenium or technetium or an isotope thereof and L is a ligand, for the preparation of a chemotoxic and optionally radiotherapeutic medicament for the treatment of cancer.
2. Use as defined in clause 1, wherein the medicament is chemotoxic by causing intrastrand linkages in DNA.
3. Use as defined in clause 1 or 2, wherein at least one of L is not; OH₂.
4. Use as defined in any one of the clauses 1-3, wherein the tricarbonyl compounds are of the general formula: 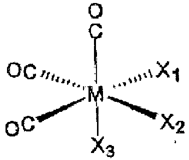 wherein M is rhenium -or technetium or an isotope thereof; at least one of X1, X2 and X3 is a monodentate ligand; or two of X1, X2 and X3 are part of a bidentate Ligand and the other one is optionally a monodentate ligand
5. Use as defined in clause 4, wherein the monodentate ligand is selected from the group consisting of halogens, CO, aromatic heterocycles, thioethers, isocyanides.
6. Use as defined in clause 5, wherein the halogens are selected from the group consisting of bromo, iodo, fluoro, chloro.
7. Use as defined in clause 5, wherein the aromatic heterocycles are selected from the group consisting of pyridine, pyrimidine, pyrazine, imidazole, pyrazole, triazole, tetrazole, thiazole, oxazole and organic molecules having one of this group as an integral part.
8. Use as defined in clause 7, wherein the purine is guanine or 9-methyl guanine.
9. Use as defined in clause 5, wherein the thioethers are selected from the group consisting of linear substituted dialkyl-thioethers or cyclic thioethers such as tetrahydrothiophen and other organic molecules containing a thioether functionality as an integral part of it.
10. Use as defined in clause 5, wherein the isocyanides are selected from the group consisting of organic molecules comprising a terminal-NC group coupled to an alkyl chain optionally comprising a functionality such as a - COOH,-NH₂, - X,-SH,-OH group.
11. Use as defined in clause 5, wherein the bidentate ligand is an amino acid or dicarboxylate.
12. Use as defined in clause 11, wherein the amino acid is an anionic amino acid.
13. Use as defined in clause 11, wherein the amino acid is a non-natural α- or β-amino acid.
14. Use as defined in clause 13, wherein the non- natural amino acid is N, N-dimethyl glycine
15. Use as defined in any one of the clauses 4-14, wherein at least two of the ligands of the tricarbonyl complex shown in formula I are exchanged by guanine or guanosine after 3 days at 37°C with guanine or guanosine being present in a slight excess over rhenium or technetium.
16. Use as defined in any one of-the clauses 4-15, wherein the compound is selected from the compounds as depicted in Figure 16 and combinations thereof.
17. Use as defined in any one of the clauses 4-16, wherein X1 and/or X2 and/or X3 are coupled to a targeting moiety.
18. Use as defined in clause 17, wherein the targeting moiety is selected from the group consisting of bombesin, neurotensin, somatostatin, glucosamine, nucleosides, nuclear localizing sequence peptides (NLS-peptides) oligonucleotides, nucleus targeting molecules such as anthracyclines, acridines and other intercalators, and derivatives or analogues thereof.
19. Tricarbonyl compounds of the general formula: 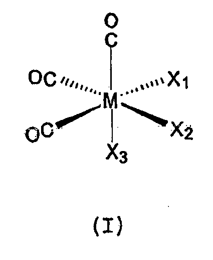 wherein M is rhenium or technetium or an isotope thereof; at least one of X1, X2 and X3 is a monodentate ligand selected from the group consisting of halogens, CO, aromatic heterocycles, thioethers, isocyanides; or two of X1, X2 and X3 are part of a bidentate ligand selected from amino acids and dicarboxylates and the other one is optionally a monodentate ligand selected from the group consisting of halogens, CO, aromatic heterocycles, thioethers, isocyanides.
20. Compounds as defined in clause 19, wherein the halogens are selected from the group consisting of bromo, iodo, fluoro, chloro.
21. Compounds as defined in-. clause 19 or 20, wherein the aromatic heterocycles are selected from the group consisting of pyridine, pyrimidine, pyrazin, imidazole, pyrazole, triazole, tetrazole, thiazole, oxazole and organic molecules having one of this group as an integral part.
22. Compounds as defined in clause 21, wherein the purine is guanine or 9-methyl guanine.
23. Compounds as defined in any one of the clauses 19- 22, wherein the thioethers are selected from the group consisting of linear substituted dialkyl-thioethers or cyclic thioethers such as tetrahydrothiophen and other organic molecules containing a thioether functionality as an integral part of it.
24. Compounds as defined in any one of the clauses 19- 23, wherein the isocyanides are selected from the group consisting of organic molecules comprising a terminal-NC group coupled to an alkyl chain optionally comprising a functionality such as a - COOH,-NH₂,-X,-SH,-OH group.
25. Compounds as defined in any one of the clauses 19- 24, wherein the bidentate ligand is an amino acid or dicarboxylate.
26. Compounds as defined in clause 25, wherein the amino acid is an anionic amino acid.
27. Compounds as defined in clause 25, wherein the amino acid is a non-natural α- or β-amino acid.
28. Compound as defined in clause 27, wherein the non- natural amino acid is N,N-dimethyl glycine.
29. Compounds as defined in any one of the clauses 19-28, wherein at least two of the ligands of the tricarbonyl complex shown in formula I are exchanged by guanine or guanosine after 3 days at 37°C with guanine or guanosine being present in a slight excess over rhenium or technetium.
30. Compound as depicted in Figure 16.
31. Compound as defined in clause 30, being complexes 6,10, 11,12, 13 and 18 as depicted in Figure 16.
32. Compounds as defined in any one of the clauses 19- 31, wherein X1 and/or X2 and/or X3 are coupled to a targeting moiety.
33. Compounds as defined in clause 32, wherein the targeting moiety is selected from the group consisting of bombesin, neurotensin, somatostatin, glucosamine, nucleosides, nuclear localizing sequence peptides (NLS-peptides), oligonucleotides, nucleus targeting molecules such as anthracyclines, acridines and other intercalators, and derivatives and analogues thereof.

## Claims

1. A metal tricarbonyl compound selected from the compounds as depicted herein below:

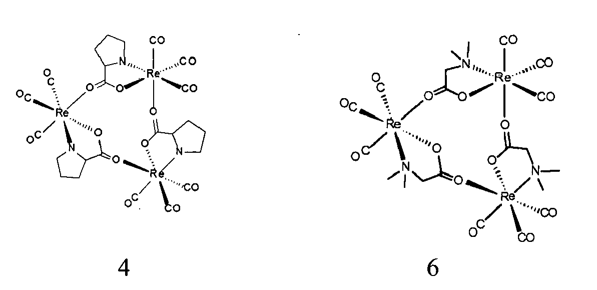

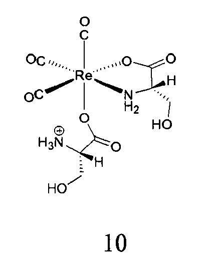

2. A compound as claimed in claim 1, wherein one or more of the ligands are coupled to a targeting moiety, wherein the targeting moiety is selected from the group consisting of bombesin, neurotensin, somatostatin, glucosamine, nucleosides, nuclear localizing sequence peptides (NLS-peptides), oligonucleotides, nucleus targeting molecules such as anthracyclines or acridines.

3. A compound as claimed in claim 1 or claim 2, for use in therapy.

4. A compound according to claim 1 or claim 2, for use in the treatment of cancer.

5. Use of a compound according to claim 1 or claim 2, for the preparation of a chemotoxic and optionally radiotherapeutic medicament for the treatment of cancer.

## Patentansprüche

1. Metall-Tricarbonyl-Verbindung ausgewählt aus den Verbindungen wie hierin nachfolgend abgebildet:

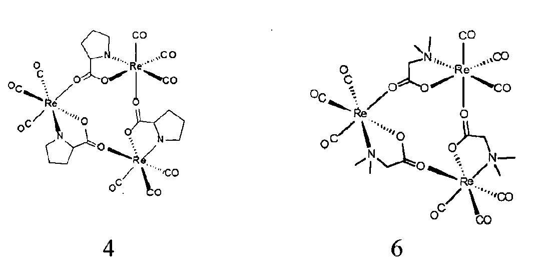

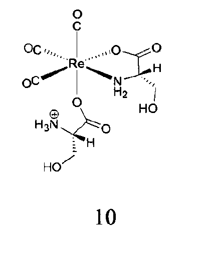

2. Verbindung wie in Anspruch 1 beansprucht, wobei ein oder mehrere der Liganden an eine zielgerichtete Gruppe gebunden sind, wobei die zielgerichtete Gruppe ausgewählt ist aus der Gruppe bestehend aus Bombesin, Neurotensin, Somatostatin, Glucosamin, Nucleosiden, Kernlokalisierungssequenz-Peptiden (NLS-Peptiden), Oligonukleotiden, auf den Zellkern gerichteten Molekülen wie beispielsweise Anthrazykline oder Acridine.

3. Verbindung wie in Anspruch 1 oder Anspruch 2 beansprucht zur Verwendung in der Therapie.

4. Verbindung gemäß Anspruch 1 oder Anspruch 2 zur Verwendung in der Behandlung von Krebs.

5. Verwendung einer Verbindung gemäß Anspruch 1 oder Anspruch 2 zur Herstellung eines chemotoxischen und wahlweise strahlentherapeutischen Medikaments zur Behandlung von Krebs.

## Revendications

1. Composé de métal tricarbonyle choisi parmi les composés tels qu'illustrés dans ce document ci-dessous :

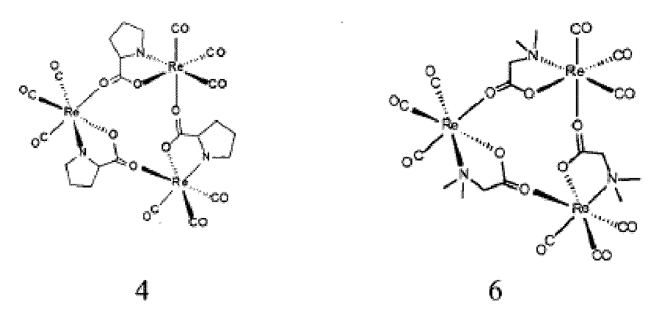

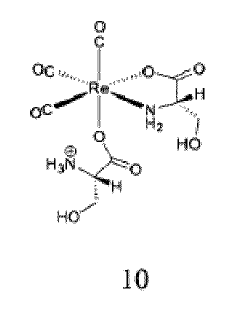

2. Composé selon la revendication 1, où un ou plusieurs des ligands sont couplés à un radical de ciblage, où le radical de ciblage est choisi parmi le groupe comprenant la bombésine, la neurotensine, la somatostatine, la glucosamine, des nucléosides, des peptides de séquence de localisation nucléaire (peptides « NLS »), des oligonucléotides, des molécules de ciblage du noyau comme des anthracyclines ou des acridines.

3. Composé selon la revendication 1 ou 2, pour une utilisation en thérapie.

4. Composé selon la revendication 1 ou 2, pour une utilisation dans le traitement du cancer.

5. Utilisation d'un composé selon la revendication 1 ou 2, pour la préparation d'un médicament chimiotoxique et éventuellement radiothérapeutique pour le traitement du cancer.
